# EUROPÄISCHE PATENTSCHRIFT

(11) **EP 2 842 579 B1**
(45) Veröffentlichungstag und Bekanntmachung des Hinweises auf die Patenterteilung: **01.06.2016**
(21) Anmeldenummer: 14183361.6
(22) Anmeldetag: 03.09.2014
(51) Int. Cl.: A61L 2/08, H01J 33/04

(54) **Verfahren und Vorrichtung zum Sterilisieren von Behältnissen mit Reinigung eines Strahlaustrittsfensters**
Method and device for sterilising containers with device for cleaning of a beam exit window
Procédé et dispositif de stérilisation de récipients avec un dispositif de nettoyage d'une fenêtre de rayon

(30) Priorität: 03.09.2013 DE 102013109584
(43) Veröffentlichungstag der Anmeldung: 04.03.2015
(73) Patentinhaber: Krones AG, 93073 Neutraubling (DE)
(72) Erfinder: Scheuren, Hans, 93073 Neutraubling (DE); Knott, Josef, 93073 Neutraubling (DE)
(74) Vertreter: Bittner, Bernhard

(56) Entgegenhaltungen:
- EP-A1- 2 161 202
- EP-A1- 2 559 444
- WO-A1-2009/144114
- WO-A1-2014/095937
- DE-U1-202012 103 519

## Beschreibung

Die vorliegende Erfindung bezieht sich auf ein Verfahren zum Betreiben einer Vorrichtung zum Sterilisieren von Behältnissen und insbesondere von Kunststoffbehältnissen sowie auf eine entsprechende Vorrichtung zum Sterilisieren von Kunststoffbehältnissen. Derartige Verfahren und Vorrichtungen sind aus dem Stand der Technik seit langem bekannt. Während man in früherer Zeit üblicherweise eine derartige Sterilisation durch die Verwendung von chemischen Substanzen, wie beispielsweise Wasserstoffperoxid oder Peressigsäure, durchgeführt hat, ist man in jüngerer Zeit bestrebt, den Einsatz derartiger Chemikalien zurückzudrängen. Daher sind aus dem Stand der Technik auch Verfahren bekannt, bei denen Kunststoffbehältnisse durch Bestrahlung, beispielsweise durch Bestrahlung mit Röntgen- oder UV-Strahlung oder auch neuerdings durch Bestrahlung mit Ladungsträgern, wie insbesondere Elektronen, sterilisiert werden.

WO 2009/144114 A1 offenbart eine Vorrichtung zum Sterilisieren von Behältnissen mit einem Behandlungskopf, der ein Austrittsfenster aufweist, durch welches hindurch Ladungsträger hindurchtreten können, mit einer Ladungsträgererzeugungsquelle, die Ladungsträger erzeugt und einer oberhalb des Austrittsfensters angeordneten Beschleunigungseinrichtung, welche die Ladungsträger in Richtung des Austrittsfensters beschleunigt und mit einer Kühleinrichtung zum Kühlen des Austrittsfensters.

US 2011/012032 offenbart eine Elektronenstrahl-Desinfektionsvorrichtung für Kunststoffbehälter.

Problematisch ist dabei insbesondere die Innensterilisation von Behältnissen, das heißt die Sterilisation der Innenwandung derartiger Kunststoffbehältnisse. Hierfür ist es bekannt, dass Strahlfinger in das Innere derartiger Kunststoffbehältnisse eingeführt werden und dort die Innenwandung der Kunststoffbehältnisse mit den Elektronen beaufschlagen.

Zu diesem Zweck weisen derartige Vorrichtungen Elektronen- oder Ladungsträgererzeugungseinrichtungen auf und üblicherweise auch Beschleunigungseinrichtungen, welche diese Ladungsträger, insbesondere innerhalb einer Vakuumkammer, in Richtung eines Austrittsfensters beschleunigen. Durch dieses Austrittsfenster, bei dem es sich beispielsweise

um ein Titanfenster handeln kann, können die Ladungsträger wiederum austreten. In einem Dauerbetrieb derartiger Strahlvorrichtungen wurde jedoch herausgefunden, dass diese Strahlvorrichtungen mit der Zeit Leistungsabfällen unterworfen sind.

Der vorliegenden Erfindung liegt daher die Aufgabe zugrunde, Möglichkeiten zu schaffen, welche die Leistungsfähigkeit derartiger Strahlfinger auch über längere Zeiträume aufrecht erhalten. Diese Aufgabe wird erfindungsgemäß durch die Gegenstände der unabhängigen Ansprüche erreicht. Vorteilhafte Ausführungsformen und Weiterbildungen sind Gegenstand der Unteransprüche.

Bei einem erfindungsgemäßen Verfahren zum Betreiben einer Vorrichtung zum Sterilisieren von Kunststoffbehältnissen werden in einem Arbeitsbetrieb Ladungsträger erzeugt und durch eine Leitungskammer transportiert. Weiterhin werden diese Ladungsträger über ein diese Leitungskammer begrenzendes Austrittsfenster aus dieser Leitungskammer austreten und das Austrittsfenster wird während der Sterilisation der Kunststoffbehältnisse wenigstens teilweise und bevorzugt auch wenigstens zeitweise durch eine Mündung der Kunststoffbehältnisse in diese eingeführt.

Erfindungsgemäß wird in einem Reinigungsbetrieb der Vorrichtung das Austrittsfenster mit einem fließfähigen Reinigungsmedium beaufschlagt bzw. diesem ausgesetzt. Es konnte ermittelt werden, dass ein Grund für den Leistungsabfall derartiger Strahlungseinrichtungen darin besteht, dass die Strahlungseinrichtungen im Dauerbetrieb einen wachsenden Schmutzbelag aufweisen. Die Verschmutzung erklärt sich aus Wechselwirkungen der aus dem Austrittsfenster austretenden Ladungsträger mit der Umgebungsluft. Die Reaktionsprodukte lagern sich entsprechend an dem Austrittsfenster ab. Bei diesen Produktionsprodukten kann es sich beispielsweise um Silikatverbindungen handeln.

Daher liegt der Erfindung die Idee zugrunde in insbesondere periodischen Abständen eine Reinigung dieses Austrittsfensters durchzuführen. Diese Reinigung findet bevorzugt zu anderen Zeiten statt als der Arbeitsbetrieb. Bevorzugt wird daher die Reinigung der Austrittsfenster außerhalb eines Arbeitsbetriebs durchgeführt. Die Reinigung kann dabei manuell, teilweise automatisch oder auch vollautomatisch durchgeführt werden.

Bevorzugt werden die besagten Austrittsfenster zum Sterilisieren in Behältnisse eingefahren. Insbesondere handelt es sich bei den Kunststoffbehältnissen um Kunststoffvorformlinge, welche bevorzugt zur Herstellung von Kunststoffflaschen dienen. Vorteilhaft weist die Vorrichtung einen stangenartigen Körper auf, der in das Innere der Kunststoffvorformlinge einführbar ist. Vorteilhaft ist dabei die besagte Leitungskammer wenigstens teilweise innerhalb dieses stangenartigen Körpers ausgebildet.

Vorteilhaft ist das besagte Austrittsfenster in einem Endabschnitt dieses Strahlfingers angeordnet.

Bei einer weiteren vorteilhaften Ausführungsform ist eine Antriebseinrichtung vorgesehen, um die besagten Austrittsfenster, insbesondere im Rahmen des Reinigungsbetriebs, in ein Behältnis mit einem Reinigungsmedium einzufahren. Vorteilhaft handelt es sich bei dem Reinigungsmedium um ein flüssiges Reinigungsmedium. Das Reinigungsmedium befindet sich (insbesondere während des Reinigungsvorgangs) vorzugsweise innerhalb des Kunststoffbehältnisses/Kunststoffvorformlings. Dies bedeutet, dass ein Kunststoffbehältnis und insbesondere ein Kunststoffbehältnis aus der Gattung derjenigen Kunststoffbehältnisse, welche sterilisiert werden sollen, in dem Reinigungsbetrieb als Aufnahmebehältnis zur Aufnahme des Reinigungsmediums verwendet wird.

Weiterhin kann es sich um die gleiche Antriebseinrichtung handeln, welche auch während des Arbeitsbetriebes die Relativbewegung zwischen den Behältnissen und dem Austrittsfenster erzeugt.

Bei einem weiteren bevorzugten Verfahren erfolgt die Erzeugung der Ladungsträger während des Sterilisationsbetriebs außerhalb des Kunststoffvorformlings. Damit werden bevorzugt zunächst die Ladungsträger erzeugt, bevorzugt mittels einer Beschleunigungseinrichtung beschleunigt und treten dann (innerhalb des Kunststoffbehältnisses) durch das Austrittsfenster aus. Das Austreten der Ladungsträger findet insbesondere bereits vor dem Eintauchen des Strahlfingers bzw. des stangenartigen Körpers in das Kunststoffbehältnis statt. Hierdurch ist es möglich, dass der obere Mündungsbereich bzw. der gesamte Gewindebereich des Kunststoffvorformlings sterilisiert wird.

Bei einem weiteren bevorzugten Verfahren wird das Austrittsfenster während des Arbeitsbetriebs wenigstens zeitweise durch Beaufschlagung mit einem gasförmigen Medium gekühlt.

Bei einem weiteren vorteilhaften Verfahren wird vor dem Beginn des Reinigungsbetriebs wenigstens ein Element einer Einrichtung zum Kühlen des Austrittsfensters von der Leitungskammer entfernt. Bei diesem weiteren Element kann es sich beispielsweise um ein Rohr handeln, welches diese Leitungskammer umgibt. Vorteilhaft wird wenigstens abschnittsweise in einer Umfangsrichtung dieser Leitungskammer ein Luftspalt ausgebildet, durch welchen hindurch Kühlluft geleitet wird. Vorteilhaft wird dieser Luftspalt zwischen der Leitungskammer, insbesondere einer Außenwandung der Leitungskammer und dem besagten Rohr gebildet.

Daneben wäre es auch denkbar, dass ein fließfähiges Reinigungsmedium über die hier beschriebene Kühlluftzuführung zugeführt wird. In diesem Fall wird bevorzugt ein gasförmiges Reinigungsmedium über die Kühlluftzuführung zugeführt. Auch wäre es möglich, dass eine Reinigung sowohl mit einem gasförmigen als auch mit einem flüssigen Reinigungsmedium durchgeführt wird. Dabei könnten diese Reinigungsmedien zeitlich versetzt oder auch (zumindest teilweise) gleichzeitig zugeführt werden. Allgemein kann es sich damit bei dem fließfähigen Reinigungsmedium auch um ein gasförmiges Reinigungsmedium handeln.

Mit anderen Worten kann in einem ersten Schritt für den Reinigungsprozess das besagte Kühlrohr, das heißt das weitere Element, abgenommen, beispielsweise abgeschraubt, werden, sodass nunmehr das eigentliche Strahlrohr, das heißt die Leitungskammer mit dem Austrittsfenster freiliegt.

Bei einem weiteren vorteilhaften Verfahren wird das Austrittsfenster zu dessen Reinigung mit wenigstens zwei unterschiedlichen fließfähigen Medien beaufschlagt bzw. zwei Medien ausgesetzt. Vorteilhaft wird das Austrittsfenster zu dessen Reinigung mit wenigstens drei unterschiedlichen Medien beaufschlagt und besonders bevorzugt mit wenigstens vier unterschiedlichen Medien.

Bei einem weiteren vorteilhaften Verfahren wird das Austrittsfenster zu dessen Reinigung in ein Flüssigkeitsbad eingetaucht. Vorteilhaft handelt es sich dabei um ein Bad der Reinigungsflüssigkeit. Vorteilhaft weisen die besagten Austrittsfenster eine Dicke in einem Bereich von 10 µm auf. Daher besteht bei einer mechanischen Reinigung in irgendeiner Form die Gefahr, dass die besagte Titanfolie hierdurch verletzt bzw. durchstoßen wird. Es wird damit eine chemische Reinigung vorgeschlagen, bei der die Titanfolie bzw. deren Außenoberfläche in ein chemisches Reinigungsmittel eingetaucht wird.

Bei einem weiteren vorteilhaften Verfahren handelt es sich bei wenigstens einem Reinigungsmedium um eine Säure. Bei einem weiteren bevorzugten Verfahren handelt es sich bei wenigstens einem Reinigungsmedium um eine Lauge.

So ist es beispielsweise möglich, dass zunächst das Austrittsfenster mit einer Säure beaufschlagt wird, anschließend mit Wasser, insbesondere mit destilliertem Wasser, anschließend mit einer Lauge und schließlich nochmals mit Wasser und insbesondere mit sterilisiertem Wasser.

Für einen vollständigen Reinigungsvorgang wird vorgeschlagen, dass zunächst die Strahleinrichtung abgeschaltet wird, anschließend die Kühlluft abgeschaltet wird, in einem weiteren Schritt das besagte Kühlrohr bzw. das weitere Element abgenommen wird, weiterhin die oben beschriebene Beaufschlagung mit den unterschiedlichen Medien erfolgt und in einem weiteren Schritt kann wiederum das Kühlrohr anmontiert werden und in einem weiteren Schritt zunächst die Kühlluft zugeschaltet wird. Anschließend wird zu einem vorgegebenen Zeitraum gewartet und schließlich kann wieder mit der Produktionsvorbereitung bzw. mit der Sterilisationsvorbereitung für Behältnisse begonnen werden.

Bei einem weiteren vorteilhaften Verfahren wird das Austrittsfenster von wenigstens einem Reinigungsmedium während eines Zeitraums beaufschlagt, der größer ist als 5 Minuten. Bevorzugt ist der Zeitraum größer als 10 Minuten, bevorzugt größer als 20 Minuten, bevorzugt größer als 30 Minuten, besonders bevorzugt größer als 40 Minuten und besonders bevorzugt größer als 50 Minuten. Bevorzugt wird das Austrittsfenster von mehreren Reinigungsmedien und besonders bevorzugt von allen Reinigungsmedien während eines Zeitraums beaufschlagt, der größer ist als 5 Minuten, bevorzugt größer als 10 Minuten, bevorzugt größer als 20 Minuten, bevorzugt größer als 30 Minuten, besonders bevorzugt größer als 40 Minuten und besonders bevorzugt größer als 50 Minuten.

Die Anmelderin hat herausgefunden, dass zu einer besonders effizienten Reinigung des Austrittsfensters dieses über einen längeren Zeitraum hinweg den jeweiligen Reinigungssubstanzen ausgesetzt werden sollte.

Bei einem weiteren vorteilhaften Verfahren wird das Austrittsfenster in ein das Reinigungsmedium enthaltendes Behältnis eingefahren. Auf diese Weise kann in besonders einfacher Weise eine effiziente Reinigung der Außenoberfläche erreicht werden. Vorteilhaft kann es sich bei diesem Behältnis um den gleichen Typ Behältnis handeln, der auch durch die Anlage sterilisiert wird. Dabei wäre es möglich, dass der stangenartige Körper bzw. das Austrittsfenster aktiv bewegt und in das Behältnis eingefahren wird. Es wäre jedoch auch möglich, und dies bevorzugt, dass das Behältnis bewegt wird und gewissermaßen über das Austrittsfenster gestülpt wird.

Bei einem weiteren vorteilhaften Verfahren wird ein an dem Austrittsfenster anliegendes Gas vor oder während des Reinigungsbetriebs entfernt. Während des Eintauchens des Austrittsfensters kann sich an diesem eine Luftblase bilden. Dabei ist zu berücksichtigen, dass bevorzugt das Austrittsfenster gegenüber einem dieses tragenden Gehäuses ein wenig zurückversetzt werden kann. Auf diese Weise kann sich beim Eintauchen die besagte Gasblase bzw. Luftblase bilden. Dieses Entfernen der besagten Luftblase erfolgt bevorzugt dadurch, dass das Austrittsfenster bzw. der besagte Strahlfinger zumindest kurzzeitig in eine Bewegung versetzt wird, beispielsweise mit einem Finger angetippt wird.

Wie oben erwähnt, wirken die einzelnen Reinigungsmedien über längere Zeiträume ein, beispielsweise zwischen ein und sechs Stunden. Es ist jedoch auch möglich, die Einwirkzeiten für einzelne Reinigungsmedien individuell zu variieren.

Der Einfluss der Reinigung konnte anhand eines Anstieges des Emissionsstroms verifiziert werden.

Vorteilhaft werden im Rahmen eines Sterilisationsvorgangs der Kunststoffbehältnisse sowohl Außen- als auch Innenbehandlungen vorgenommen. Vorteilhaft wird dabei im Rahmen der Außenbehandlung über eine feststehende Strahlungseinrichtung und insbesondere einen feststehenden Flächenstrahler das Kunststoffbehältnis von außen entkeimt. In der Innenbehandlung werden, wie oben erwähnt, bevorzugt Fingerstrahler eingesetzt. Diese sind vorteilhaft auf einem rotierenden Karussell angeordnet und während der Drehung dieses Karussells werden vorteilhaft die zu sterilisierenden Kunststoffbehältnisse über die besagten Strahlfinger bzw. auch deren Austrittsfenster gefahren. Dabei imitieren beide Strahlungseinrichtungen Elektronen, die die bestrahlten Oberflächen entkeimen.

Es wird darauf hingewiesen, dass die hier vorgeschlagene Reinigungsmöglichkeit auch für die besagten Flächenstrahler möglich ist. In diesem Falle können ebenfalls die Austrittsfenster mit einem Reinigungsmedium und insbesondere einem Flüssigreinigungsmedium beaufschlagt werden. In diesem Falle ist es jedoch nicht erforderlich, dass die Austrittsfenster während der Sterilisation der Kunststoffbehältnisse in diese eingeführt werden. Die Anmelderin behält sich vor, allgemein das vorliegende Reinigungsverfahren auch für andere Elektronenstrahler zu beanspruchen. Das Verfahren ist jedoch besonders für die besagten Strahlerfinger geeignet, da diese ein besonders empfindliches Austrittsfenster aufweisen.

Durch das hier beschriebene Verfahren kann die Lebensdauer, insbesondere dieser Fingerstrahler, erhöht werden. Um das Durchtrennen der Austrittsfenster bzw. Fensterfolie eines derartigen Strahlers zu verhindern, wird, wie oben erwähnt, vorgeschlagen, dieses Austrittsfenster bzw. die Folie in regelmäßigen Abständen zu reinigen. Dabei werden, wie oben erwähnt, bevorzugt Behältnisse vorgesehen, die mit einem Reinigungsmittel wie beispielsweise Salzsäure und/oder Lauge gefüllt sind und welche über die zu reinigenden Fenster gefahren werden. Dabei wäre es insbesondere in Abhängigkeit von einem Verschmutzungsgrad auch möglich, die jeweiligen Reinigungsvorgänge zu wiederholen.

Durch ein regelmäßiges Reinigen, insbesondere der besagten Austrittsfenster bzw. Folien, wird die Lebensdauer eines derartigen Strahlers drastisch erhöht. Auch können die Kosten aufgrund eines minimalen Chemikalieneinsatzes gering gehalten werden. Dieser minimale Chemikalieneinsatz kann unter anderem dadurch erreicht werden, dass die besagten Strahlfinger in diejenigen Behältnisse eingetaucht werden, welche dem Typ nach auch zur Sterilisation verwendet werden, insbesondere Kunststoffvorformlinge. Der Vorteil besteht darin, dass die bereits vorhandene Vorrichtung zum Sterilisieren auch für den Reinigungsbetrieb genutzt werden kann, indem (wie auch im Reinigungsbetrieb) die Austrittsfenster in die Kunststoffvorformlinge eingefahren werden. Weiterhin ist das Innenvolumen dieser Kunststoffvorformlinge sehr gering, sodass auch in nur geringem Maße Reinigungsmittel eingesetzt werden muss. Dabei werden vorteilhaft diejenigen Elemente, die für den Arbeitsbetrieb verwendet werden, auch für den Reinigungsbetrieb genutzt.

Die vorliegende Erfindung ist weiterhin auf eine Vorrichtung zum Sterilisieren von Kunststoffbehältnissen nach Anspruch 11 gerichtet.

Vorteilhaft ist das besagte Austrittsfenster durch eine Mündung des zu sterilisierenden Kunststoffbehältnisses in dieses Kunststoffbehältnis einführbar.

Erfindungsgemäß weist die Vorrichtung eine Reinigungseinrichtung auf, welche das Austrittsfenster wenigstens zeitweise mit einem flüssigen Reinigungsmedium beaufschlagt. Vorteilhaft ist dabei diese Reinigungseinrichtung derart ausgebildet, dass ein Eintauchen des Austrittsfensters in das Reinigungsmittel erfolgen kann.

Vorteilhaft weist diese Reinigungseinrichtung eine Bewegungseinrichtung auf, welche die Austrittsfenster in das Reinigungsmedium einführt.

Bei einer weiteren vorteilhaften Ausführungsform weist die Vorrichtung eine Kühleinrichtung auf, welche zumindest in einem Arbeitsbetrieb das besagte Austrittsfenster kühlt, insbesondere indem diese Kühleinrichtung das Austrittsfenster mit einem gasförmigen Medium beaufschlagt.

Bei einer weiteren vorteilhaften Ausführungsform weist diese Kühleinrichtung ein zweites Gehäuse auf, welches bevorzugt wenigstens teilweise in einer Umfangsrichtung um die Leitungskammer ausgebildet ist. Vorteilhaft wird dabei zwischen diesem zweiten Gehäuse und der Leitungskammer ein Kanal ausgebildet, durch welchen hindurch das gasförmige Medium gefördert werden kann. Dieser Kanal wird bevorzugt ebenfalls mit Reinigungsmedium gefüllt und erlaubt bevorzugt eine alternative Zuführung von Reinigungsmedium zum Ort der Reinigung.

Bei einer bevorzugten Ausführungsform weist die Vorrichtung mehrere Sterilisationseinrichtungen auf, beispielsweise eine Außensterilisationseinrichtung, welche dazu bestimmt ist, eine Außenoberfläche der Kunststoffvorformlinge mit den Elektronen zu bestrahlen, und eine Innenbestrahlungseinrichtung, welche dazu bestimmt ist, eine innenoberfläche der Kunststoffvorformlinge mit den Elektronen zu beaufschlagen. Weiterhin kann eine Vielzahl von Transporteinrichtungen vorgesehen sein, welche die Kunststoffvorformlinge während ihrer Sterilisation transportieren wie etwa Transportsterne, welche die Kunststoffvorformlinge von einer ersten Sterilisationseinrichtung zu einer zweiten Sterilisationseinrichtung fördern.

Bevorzugt ist wenigstens eine Transporteinrichtung, welche die Kunststoffvorformlinge von einer ersten Sterilisationseinrichtung (insbesondere einer Außensterilisationseinrichtung) zu einer zweiten Sterilisationseinrichtung (insbesondere einer Innensterilisationseinrichtung) fördert bzw. transportiert, als Teilungsverzugsstern ausgeführt, der in der Lage ist, nicht nur die Kunststoffbehältnisse zu transportieren, sondern auch, eine Teilung bzw. einen Abstand zweier aufeinanderfolgender Kunststoffbehältnisse zu ändern.

Ein derartiger Teilungsverzugsstern kann dabei einen bewegbaren und insbesondere drehbaren Träger aufweisen, an dem wiederum schwenkbar und/oder radial beweglich Greifelemente zum Greifen der Kunststoffbehältnisse angeordnet sind. Vorzugsweise ist in einer Transportrichtung der Kunststoffvorformlinge zunächst eine Außensterilisationseinrichtung zum Sterilisieren einer Außenoberfläche und dann eine Innensterilisationseinrichtung zum Sterilisieren einer Innenoberfläche des Kunststoffvorformlings vorgesehen.

Bei einer weiteren vorteilhaften Ausführungsform ist das besagte zweite Gehäuse von der Leitungskammer abnehmbar. So kann beispielsweise das zweite Gehäuse an der Leitungskammer angeschraubt sein.

Bei einer vorteilhaften Ausführungsform ist das Reinigungsmittel (insbesondere während des Reinigungsvorgangs) in einem Kunststoffbehältnis aufgenommen. Bevorzugt handelt es sich dabei um ein Kunststoffbehältnis, welches hinsichtlich seiner Gattung denjenigen Kunststoffbehältnissen entspricht, welche sterilisiert werden sollen.

Bei einer weiteren vorteilhaften Ausführungsform weist die Reinigungseinrichtung ein Behältnis auf, in welches das Austrittsfenster einführbar ist. Vorteilhaft handelt es sich dabei bei dem Behältnis vom Typ her um das gleiche Behältnis, welches auch sterilisiert wird. Insbesondere handelt es sich bei dem Behältnis um einen Kunststoffvorformling.

Bei einer weiteren vorteilhaften Ausführungsform weist die Leitungskammer einen stangenartigen Körper auf, bzw. bildet einen stangenartigen Körper aus, der in den Kunststoffvorformling einführbar ist.

Bei einer weiteren vorteilhaften Ausführungsform weist die Vorrichtung einen Träger auf, an dem eine Vielzahl der oben beschriebenen Vorrichtungen und insbesondere eine Vielzahl der oben beschriebenen Strahler angeordnet sind. Vorteilhaft handelt es sich hierbei um einen drehbaren Träger.

Bei einer weiteren vorteilhaften Ausführungsform weist die Vorrichtung eine Antriebseinrichtung auf, welche ein Einführen der stangenartigen Körper bzw. der Austrittsfenster in einem Arbeitsbetrieb in die zu sterilisierenden Kunststoffbehältnisse ermöglicht.

Bei einer weiteren vorteilhaften Ausführungsform weist das Austrittsfenster eine Folie auf bzw. ist als Folie ausgebildet. Dabei handelt es sich besonders bevorzugt um eine Titanfolie. Bevorzugt weist diese Folie eine Dicke auf, welche zwischen 6 und 20 µm, bevorzugt zwischen 8 und 15 µm und besonders bevorzugt zwischen 8 und 12 µm liegt.

Bei einer weiteren vorteilhaften Ausführungsform weist die Vorrichtung eine Befüllungseinrichtung auf, welche diejenigen Behältnisse, in welche die Austrittsfenster eingetaucht werden, wenigstens teilweise mit einem Reinigungsmedium befüllt.

Weiterhin kann die Reinigungseinrichtung bevorzugt Bevorratungsbehältnisse bzw. Bevorratungseinrichtungen aufweisen, welche die besagten Reinigungsmedien bevorraten.

So ist es beispielsweise möglich, dass der besagte Reinigungsbetrieb auch automatisch durchführbar ist. Dabei ist es möglich, dass in einem Vorbereitungsgang des Reinigungsbetriebs zunächst die einzelnen Behältnisse mit einer oder mehreren Reinigungsflüssigkeiten befüllt werden. Anschließend können die Strahlfinger in diese Behältnisse eintauchen. Dabei wäre es möglich, dass im Rahmen des Reinigungsbetriebes mehrere der besagten Behältnisse mit einem ersten Reinigungsmedium befüllt werden und anschließend die Austrittsfenster für einen vorgegebenen Zeitraum in diese Behältnisse eingetaucht werden. Anschließend können die Behältnisse entleert und mit einer zweiten Reinigungsflüssigkeit befüllt werden und hier wieder die Austrittsfenster eingetaucht werden.

Damit ist es möglich, dass der Reinigungsbetrieb automatisch durchgeführt wird.

Weitere Vorteile und Ausführungsformen ergeben sich aus der beigefügten Zeichnung. Dabei zeigt:
- Fig. 1: eine Darstellung einer erfindungsgemäßen Vorrichtung; und
- Fig. 2: eine Darstellung einer erfindungsgemäßen Vorrichtung in einem Reinigungsmodus.

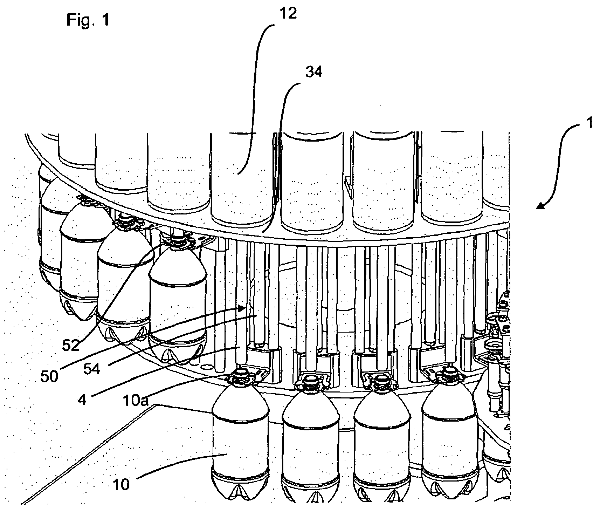

Figur 1 zeigt eine Darstellung einer erfindungsgemäßen Vorrichtung zum Sterilisieren von Behältnissen. Dabei weist diese Vorrichtung einen drehbaren Träger 34 auf, an dem eine Vielzahl von Vorrichtungen 1 zum Sterilisieren von Kunststoffbehältnissen 10 angeordnet ist. Bei den Behältnissen 10 handelt es sich hier um Kunststoffflaschen, es wird jedoch darauf hingewiesen, dass mit der erfindungsgemäßen Vorrichtung auch Kunststoffvorformlinge sterilisiert werden können und dies auch bevorzugt ist.

Das Bezugszeichen 50 kennzeichnet in seiner Gesamtheit eine Bewegungseinrichtung, welche hier die Kunststoffbehältnisse 10 bewegt, insbesondere anhebt, und so die einzelnen Strahlfinger 4 über die Mündungen der Behältnisse 10 in diese einführt. Die Bewegungseinrichtungen 50 weisen jeweils Halteeinrichtungen 52 auf, welche die Behältnisse halten und hier insbesondere unterhalb deren Tragring halten. Daneben weisen die Bewegungseinrichtungen 50 einen Stangenkörper 54 auf, der linear entlang seiner Längsrichtung bewegt werden kann, um die Behältnisse anzuheben.

Daneben weist die Vorrichtung auch bevorzugt einen Reinraum auf, innerhalb dessen die Kunststoffvorformlinge während des Arbeitsbetriebs transportiert werden. Dieser Reinraum kann dabei durch mehrere Wandungen (nicht gezeigt) begrenzt werden, wobei bevorzugt eine dieser Wandungen gegenüber einer weiteren Wandung beweglich ist.

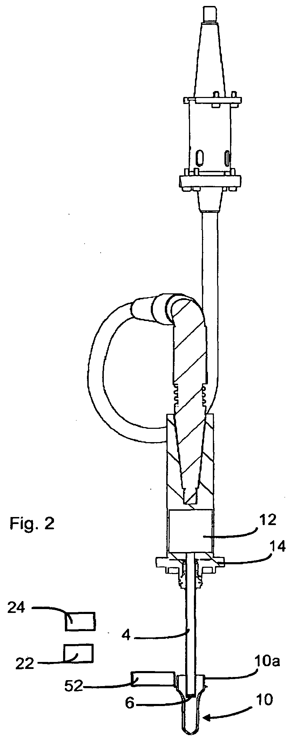

Figur 2 zeigt eine Detailansicht einer erfindungsgemäßen Vorrichtung in einem Reinigungsmodus. Dabei bezieht sich das Bezugszeichen 12 auf eine Beschleunigungskammer, in der die Elektronen beschleunigt werden. Diese Beschleunigungskammer wird dabei nicht in die Behältnisse eingeführt. An diese Beschteunigungskammer 12 schließt sich eine Kammer 4 beispielsweise in Form eines Fingers an, der in die Kunststoffbehältnisse 10 über deren Mündung 10a einführbar ist. Am unteren Ende dieses Strahlfingers ist das Austrittfenster 6 vorgesehen, über welches die Elektronen aus der Kammer 4 austreten können.

Das Bezugszeichen 22 kennzeichnet grob schematisch eine Antriebseinrichtung, welche zu Reinigungszwecken das Behältnis 10 hier nach oben hebt und damit das Austrittsfenster in den Kunststoffvorformling 10 einführt. Dabei kann es sich hier um die gleiche Antriebseinrichtung handeln, die auch im Arbeitsbetrieb die Kunststoffvorformlinge hebt und senkt.

Das Bezugszeichen 24 kennzeichnet eine Steuerungseinrichtung, welche die Bewegung der Kunststoffvorformlinge 10 in dem Reinigungsmodus steuert. Dabei ist es denkbar, dass die Kunststoffvorformlinge 10 angehoben werden und auf diese Weise die Kammer 4 mit dem Austrittsfenster 6 in die Kunststoffvorformlinge eingetaucht wird. Anschließend kann die Vorrichtung in diesem Zustand über eine längere Zeitspanne gehalten werden, um - wie oben beschrieben - die Reinigung des Austrittsfensters 6 zu bewirken.

Dabei ist es möglich, dass dieser Vorgang gleichzeitig mit allen Strahlungseinrichtungen 1, die an dem gemeinsamen Träger 34 angeordnet sind, durchgeführt wird. In einem weiteren Schritt können, wie oben beschrieben, andere Reinigungsmedien eingesetzt werden.

### Bezugszeichenliste

- 1: Vorrichtung zum Sterilisieren von Behältnissen
- 2: Ladungsträgererzeugungseinrichtung
- 4: Kammer
- 6: Austrittsfenster
- 10: Kunststoffbehältnisse, Behältnis, Kunststoffvorformling
- 10a: Mündung
- 12: Beschleunigungskammer
- 20: Reinigungseinrichtung
- 22: Antriebseinrichtung
- 24: Steuerungseinrichtung
- 34: Träger
- 50: Bewegungseinrichtung
- 52: Halteeinrichtung
- 54: Stangenkörper

## Patentansprüche

1. Verfahren zum Betreiben einer Vorrichtung (1) zum Sterilisieren von Kunststoffbehältnissen (10), wobei in einem Arbeitsbetrieb Ladungsträger erzeugt und durch eine Leitungskammer (4) transportiert werden und über ein diese Leitungskammer (4) begrenzendes Austrittsfenster (6) aus dieser Leitungskammer (4) austreten und wobei das Austrittsfenster (6) während der Sterilisation der Kunststoffbehältnisse (10) wenigstens teilweise durch eine Mündung (10a) der Kunststoffbehältnisse (10) in diese eingeführt wird, ,
**dadurch gekennzeichnet, dass**
in einem Reinigungsbetrieb der Vorrichtung das Austrittsfenster (6) mit einem flüssigen Reinigungsmedium beaufschlagt wird, wobei das Austrittsfenster (6) in ein das Reinigungsmedium enthaltendes Behältnis (10) eingefahren wird.

2. Verfahren nach Anspruch 1,
**dadurch gekennzeichnet, dass**
das Austrittsfenster (6) während des Arbeitsbetriebs wenigstens zeitweise durch Beaufschlagung mit einem gasförmigen Medium gekühlt wird.

3. Verfahren nach wenigstens einem der vorangegangenen Ansprüche,
**dadurch gekennzeichnet, dass**
vor dem Beginn des Reinigungsbetriebs wenigstens ein Element einer Einrichtung zum Kühlen des Austrittsfensters (6) von der Leitungskammer (4) entfernt wird.

4. Verfahren nach wenigstens einem der vorangegangenen Ansprüche,
**dadurch gekennzeichnet, dass**
das Austrittsfenster (6) zu dessen Reinigung mit wenigstens zwei unterschiedlichen flüssigen Medien beaufschlagt wird.

5. Verfahren nach wenigstens einem der vorangegangenen Ansprüche,
**dadurch gekennzeichnet, dass**
das Austrittsfenster (6) zu dessen Reinigung in ein Flüssigkeitsbad eingetaucht wird.

6. Verfahren nach wenigstens einem der vorangegangenen Ansprüche,
**dadurch gekennzeichnet, dass**
wenigstens ein Reinigungsmedium eine Säure ist.

7. Verfahren nach wenigstens einem der vorangegangenen Ansprüche,
**dadurch gekennzeichnet, dass**
wenigstens ein Reinigungsmedium eine Lauge ist.

8. Verfahren nach wenigstens einem der vorangegangenen Ansprüche,
**dadurch gekennzeichnet, dass**
das Austrittsfenster (6) von wenigstens einem Reinigungsmedium während eines Zeitraums beaufschlagt wird, der größer ist als 5 min.

9. Verfahren nach wenigstens einem der vorangegangenen Ansprüche,
**dadurch gekennzeichnet, dass**
ein an dem Austrittsfenster (6) anliegendes Gas vor oder während des Reinigungsbetriebs entfernt wird.

10. Vorrichtung (1) zum Sterilisieren von Kunststoffbehältnissen (10) mit einer Ladungsträgererzeugungseinrichtung (2), welche Ladungsträger erzeugt, mit einer Leitungskammer (4) zum Leiten der von der Ladungsträgererzeugungseinrichtung erzeugten Ladungsträger und mit einem Austrittsfenster (6) durch welches hindurch die Ladungsträger aus der Leitungskammer (4) austreten können, wobei dieses Austrittsfenster (6) durch eine Mündung des zu sterilisierenden Kunststoffbehältnisses (10) in das Kunststoffbehältnis (10) einführbar ist, wobei die Vorrichtung (1) eine Reinigungseinrichtung (20, 50, 52, 54) aufweist, welche das Austrittsfenster (6) wenigstens zeitweise mit einem flüssigen Reinigungsmittel beaufschlagt,
**dadurch gekennzeichnet, dass** die Vorrichtung (1) eine Steuerungseinrichtung (24) aufweist, welche zur Durchführung des verfahrens nach Anspruch 1 ansgebildet ist.

11. Vorrichtung (1) nach Anspruch 10,
**dadurch gekennzeichnet, dass**
ein Kunststoffbehältnis (10) in einem Reinigungsbetrieb als Aufnahmebehältnis zur Aufnahme des Reinigungsmediums verwendbar ist.

12. Vorrichtung (1) nach Anspruch 11,
**dadurch gekennzeichnet, dass**
das Kunststoffbehältnis (10), welches in dem Reinigungsbetrieb als Aufnahmebehältnis des Reinigungsmediums verwendbar ist, aus der Gattung derjenigen Kunststoffbehältnisse (10) ist, welche sterilisiert werden sollen.

13. Vorrichtung (1) nach Anspruch 10,
**dadurch gekennzeichnet, dass**
die Vorrichtung (1) eine Befüllungseinrichtung aufweist, welche diejenigen Behältnisse (10), in welche die Austrittsfenster (6) eingetaucht werden, wenigstens teilweise mit einem Reinigungsmedium befüllt.

14. Vorrichtung (1) nach Anspruch 10,
**dadurch gekennzeichnet, dass**
die Vorrichtung (1) mehrere Sterilisationseinrichtungen aufweist und eine Vielzahl von Transporteinrichtungen, welche die Kunststoffvorformlinge (10) während ihrer Sterilisation transportieren, welche die Kunststoffvorformlinge (10) von einer ersten Sterilisationseinrichtung zu einer zweiten Sterilisationseinrichtung fördem, wobei wenigstens eine Transporteinrichtung, welche die Kunststoffvorformlinge (10) von einer ersten Sterilisationseinrichtung zu einer zweiten Sterilisationseinrichtung transportiert, als Teilungsverzugsstern ausgeführt, der in der Lage ist, nicht nur die Kunststoffbehältnisse (10) zu transportieren, sondern auch, eine Teilung bzw. einen Abstand zweier aufeinanderfolgender Kunststoffbehältnisse (10) zu ändern.

## Claims

1. A method of operating an apparatus for the sterilization of plastics material containers, wherein charge carriers are generated in a working operation and are conveyed through a guide chamber (4) and emerge from this guide chamber (4) by way of an outlet window (6) bounding this guide chamber (4) and wherein the outlet window (6) is introduced into the plastics material containers (10) at least in part through an aperture (10a) of the aforesaid plastics material containers during the sterilization of the plastics material containers (10), **characterized in that** the outlet window (6) is acted upon with a liquid cleaning medium in a cleaning operation of the apparatus, wherein the outlet window (6) is introduced in a container (10) containing the cleaning medium.

2. A method according to claim 1, **characterized in that** the outlet window (6) is cooled at least for a time by being acted upon with a gaseous medium during the working operation.

3. A method according to at least one of the preceding claims, **characterized in that** at least one element of a device for cooling the outlet window (6) is removed from the guide chamber (4) before the beginning of the cleaning operation.

4. A method according to at least one of the preceding claims, **characterized in that** the outlet window (6) is acted upon with at least two different flowable media for the cleaning thereof.

5. A method according to at least one of the preceding claims, **characterized in that** the outlet window (6) is dipped into a bath of liquid for its cleaning.

6. A method according to at least one of the preceding claims, **characterized in that** at least one cleaning medium is an acid.

7. A method according to at least one of the preceding claims, **characterized in that** at least one cleaning medium is a lye.

8. A method according to at least one of the preceding claims, **characterized in that** the outlet window (6) is acted upon by at least one cleaning medium for a period of time which is greater than 5 minutes.

9. A method according to at least one of the preceding claims, **characterized in that** a gas present at the outlet window (6) is removed before or during the cleaning operation.

10. An apparatus (1) for the sterilization of plastics material containers (10), with a charge carrier generation device (2) which generates charge carriers, with a guide chamber (4) for guiding the charge carriers generated by the charge carrier generation device, and with an outlet window (6) through which the charge carriers can emerge out of the guide chamber (4), wherein this outlet window (6) is capable of being introduced into the plastics material container (10) through an aperture of this plastics material container to be sterilized, wherein the apparatus has a cleaning device (20, 50, 52, 54) which acts upon the outlet window (6) with a liquid cleaning medium at least for a time, **characterized in that** the apparatus (1) has a control device (24) which is designed for carrying out the method according to claim 1.

11. An apparatus (1) according to claim 10,
**characterized in that**
a plastics material container can be used during cleaning operation as a receiving container for receiving the cleaning medium.

12. An apparatus (1) according to claim 11,
**characterized in that**
the plastics material container, which can be used as a receiving container of the cleaning medium during cleaning operation is the kind of plastics material containers (10) which are to be sterilized.

13. An apparatus (1) according to claim 10,
**characterized in that**
the apparatus (1) has a filling device which fills the containers (10) into which the outlet windows (6) are dipped at least in part with a cleaning medium.

14. An apparatus (1) according to claim 10,
**characterized in that**
the apparatus (1) comprises a plurality of sterilization devices and of transport devices which transport the plastics material preforms (10) during the sterilization thereof, which convey the plastics material preforms (10) from a first sterilization device to a second sterilization device, wherein at least one transport device, which transports the plastics material preforms (10) from a first sterilization device to a second sterilization device is designed as a distribution star which is not only able to transport the plastics material containers (10) but also to change a partition or a distance respectively of two plastics material containers (10) subsequent with respect to each other.

## Revendications

1. Procédé de fonctionnement d'une installation (1) pour la stérilisation de récipients en matière plastique (10), dans lequel, dans un mode de travail, des porteurs de charge sont produits et transportés à l'intérieur d'un compartiment de conduite (4), et sortent dudit compartiment de conduite (4) par une fenêtre de sortie (6) délimitant ledit compartiment de conduite (4), et dans lequel la fenêtre de sortie (6) est au moins partiellement introduite par une embouchure (10a) des récipients en matière plastique (10) dans les récipients en matière plastique (10) pendant la stérilisation des récipients en matière plastique (10),
**caractérisé en ce que**,
dans un mode de nettoyage de l'installation, un fluide de nettoyage liquide est appliqué sur la fenêtre de sortie (6), la fenêtre de sortie (6) étant plongée dans un récipient (10) contenant le fluide de nettoyage.

2. Procédé selon la revendication 1,
**caractérisé en ce que**
la fenêtre de sortie (6) est pendant le mode de travail au moins temporairement refroidie par application d'un fluide gazeux.

3. Procédé selon au moins une des revendications précédentes,
**caractérisé en ce**
**qu'**avant le début du mode de nettoyage, au moins un élément d'un dispositif pour le refroidissement de la fenêtre de sortie (6) est retiré du compartiment de conduite (4).

4. Procédé selon au moins une des revendications précédentes,
**caractérisé en ce**
**qu'**au moins deux fluides liquides différents sont appliqués sur la fenêtre de sortie (6) pour le nettoyage de celle-ci.

5. Procédé selon au moins une des revendications précédentes,
**caractérisé en ce que**
pour être nettoyée, la fenêtre de sortie (6) est plongée dans un bain de liquide.

6. Procédé selon au moins une des revendications précédentes,
**caractérisé en ce**
**qu'**au moins un fluide de nettoyage est un acide.

7. Procédé selon au moins une des revendications précédentes,
**caractérisé en ce**
**qu'**au moins un fluide de nettoyage est une lessive.

8. Procédé selon au moins une des revendications précédentes,
**caractérisé en ce**
**qu'**au moins un fluide de nettoyage est appliqué sur la fenêtre de sortie (6) pendant une période supérieure à 5 min.

9. Procédé selon au moins une des revendications précédentes,
**caractérisé en ce**
**qu'**un gaz présent sur la fenêtre de sortie (6) est retiré avant ou pendant le mode de nettoyage.

10. Installation (1) pour la stérilisation de récipients en matière plastique (10), avec un dispositif générateur de porteurs de charge (2) générant des porteurs de charge, avec un compartiment de conduite (4) pour le transport des porteurs de charge générés par le dispositif générateur de porteurs de charge et avec une fenêtre de sortie (6) par laquelle les porteurs de charge peuvent sortir du compartiment de conduite (4), ladite fenêtre de sortie (6) pouvant être introduite dans le récipient en matière plastique (10) par une embouchure dudit récipient en matière plastique (10) à stériliser, ladite installation (1) comprenant un dispositif de nettoyage (20, 50, 52, 54) qui applique au moins temporairement un produit de nettoyage liquide sur la fenêtre de sortie (6),
**caractérisée en ce que**
ladite installation (1) comprend un dispositif de commande (24) prévu pour l'exécution du procédé selon la revendication 1.

11. Installation (1) selon la revendication 10,
**caractérisée en ce**
**qu'**un récipient en matière plastique (10) est dans un mode de nettoyage prévu comme récipient de collecte pour le fluide de nettoyage.

12. Installation (1) selon la revendication 11,
**caractérisée en ce que**
le récipient en matière plastique (10) utilisable comme récipient de collecte du fluide de nettoyage en mode de nettoyage est du même type que les récipients en matière plastique (10) devant être stérilisés.

13. Installation (1) selon la revendication 10,
**caractérisée en ce que**
ladite installation (1) comprend un dispositif de remplissage qui remplit au moins partiellement avec un fluide de nettoyage les récipients (10) où sont plongées les fenêtres de sortie (6).

14. Installation (1) selon la revendication 10,
**caractérisée en ce que**
ladite installation (1) comprend plusieurs dispositifs de stérilisation et une pluralité de dispositifs de transport qui convoient les préformes en matière plastique (10) pendant leur stérilisation et qui convoient les préformes en matière plastique (10) d'un premier dispositif de stérilisation à un deuxième dispositif de stérilisation, au moins un dispositif de transport convoyant les préformes en matière plastique (10) d'un premier dispositif de stérilisation à un deuxième dispositif de stérilisation étant réalisé sous forme d'étoile de retardement/distribution apte non seulement à transporter les récipients en matière plastique (10), mais aussi à modifier un pas ou un espacement respectivement entre deux récipients en matière plastique (10) consécutifs.
